# EUROPEAN PATENT SPECIFICATION

(11) **EP 0 599 661 B1**
(45) Date of publication and mention of the grant of the patent: **01.10.1997**
(21) Application number: 93309468.2
(22) Date of filing: 26.11.1993
(51) Int. Cl.: C05F 17/02, A47K 11/02

(54) **Fermentation treatment apparatus of organic refuse possessing malodor release preventive function**
Fermentationsvorrichtung für organische Abfälle zur Verhinderung von Geruchtsabgabe
Appareil de fermentation de déchets organiques avec prévention du dégagement des mauvaises odeurs

(30) Priority: 26.11.1992 JP 341457/92; 30.06.1993 JP 189056/93
(43) Date of publication of application: 01.06.1994
(73) Proprietor: Shimizu, Hiroshi, Morioka-shi, Iwate 020-01 (JP); OTSUKA SCIENCE CO., LTD., Nagoya-shi, Aichi 467 (JP)
(72) Inventor: Shimizu, Hiroshi, Morioka-shi, Iwate 020-01 (JP)
(74) Representative: W.P. Thompson & Co.

(56) References cited:
- EP-A- 0 085 000
- EP-A- 0 434 159
- WO-A-90/00162
- WO-A-91/12829
- WO-A-92/18611
- US-A- 2 878 112
- US-A- 5 204 263

## Description

The present invention relates to a fermentation treatment apparatus of organic refuse for fermenting and drying organic refuse of relatively high water content such as domestic refuse, night soil, livestock waste, food processing plant refuse, and water treatment excess sludge, hygienically and efficiency without release malodor, and utilizing as organic fertilizer in powder form.

The technology of fermentation treatment of organic waste is long, but hitherto fermentation was unstable and always accompanied by violating malodor. In particular, in fermentation treatment of organic refuse of high moisture content, the charging material itself often releases malodor, and when treated by fermentation heat, it is chemically decomposed, it often produces organic acids with a strong offensive smell. Or, if the treating container is heated by heater to enhance the processing efficiency of the apparatus, generation of malodor is further increased.

Such malodor often induces pollution problems. To solve these problems, it was proposed to furnish the treating apparatus with a deodorizing device, but it requires an additional cost and causes an increase in the size of the apparatus, and it was difficult to maintain the function for a long time.

On the other hand, to ferment actively an organic refuse of high water content, it is necessary to grind the charging material sufficiently to increase the reaction surface area, ferment and dry enough to mix with mature matter of low water content rich in active bacteria, and maintain the mixture in a moisture condition and a permeable porous condition necessary for bacterial digestion. By cutting and grinding by mixer and disposer, the organic refuse becomes small grains, and from the viewpoint of the cell tissues, the increase of reaction surface area is insufficient, and the organic matter in the particles is in anaerobic condition same as in the prior art of charging the material directly without grinding, and it leads to release of malodor due to temperature elevation by fermentation.

WO-A-9000162, US-A-2878112, EP-A-85000 and WO-A-9112829 disclose apparatus for fermenting organic matter. However, they suffer from a number of disadvantages. For example, the prior art arrangements have no means for agitating the matter to distribute water evenly. Thus, if the organic matter to be treated contains a large amount of water, aerobic fermentation might be suppressed and anaerobic fermentation might be encouraged. Also, the prior art arrangements have no means of controlling the height of the organic matter, which makes it more difficult to control the aerobic fermentation.

It is hence a primary object of the invention to present a fermentation treatment apparatus of organic refuse processing a high treating capacity, by preventing generation of smell occurring in the fermentation treatment process.

It is another object of the invention to present a fermentation treatment apparatus of organic refuse characterized by preliminarily increasing the reaction surface area of organic refuse to be processed by fermentation.

In accordance with the present invention, there is provided a fermentation treatment apparatus for organic refuse comprising:
(a) an enclosed container having;
   (i) a charging port from which organic refuse is fed to form a stacked layer in said container and
   (ii) a discharge port spaced apart from said charging port;
(b) an agitating means for laterally transferring said stacked layer, while agitating the stacked layer vertically, from said charging port to the discharge port in said container, to continuously form a main fermentation region and a ripening region; and
(c) an exhaust means for exhausting gas generated in said stacked layer outside said container through said upper space; characterised by
(d) a stack height-holding means for holding a height of said stacked layer to form an upper space in said container; and
(e) an air supply means on a bottom of said container for supplying air to said stacked layer.

In one embodiment, the apparatus further comprises a sucking and ventilating means having:
an aspiration tube for sucking odorant components generated in said stacked layer in a vicinity of said charging port;
a ventilating means for ventilating said odorant components to said stacked layer, and
a gas pumping means for transferring said odorant components from said aspiration tube to said ventilating means.

By introducing the gas containing malodor mainly originated from a pre-fermentation region and its vicinity into the stacked layer, digestion by microorganisms is progressed, so that generation of malodor may be prevented. Therefore, fermentation treatment of organic refuse can be easily and economically executed even in a crowded district. The obtained fermented product can be used also as organic fertilizer.

Moreover, the fermentation treatment apparatus brings about the effect of increasing the function itself of fermentation treatment. That is, by dividing and aligning the pre-fermentation region, main fermentation region, and ripening region in the apparatus, the optimum fermentation condition suited to each stage can be given.

It is preferred to return a part of mature matter from the ripening chamber to the grinding chamber to mix with ground material.

Crushing under grinding action in the invention contains an embodiment of grinding action compensated by velocity, such as hammer mill, which is useful to the low compaction condition, as well as sliding rotation of the transfer shaft against the friction cylinder.

When composing the grinding chamber by installing the conveying shaft in the center of friction cylinder, it is desired to set the sectional area in the friction cylinder sufficiently smaller than the sectional area of the main fermentation chamber, for example, 1/2 to 1/100.

By way of example only, specific embodiments of the present invention will now be described with reference to the accompanying drawings, in which:-
Fig. 1 is a longitudinal sectional view showing a first embodiment of the invention;
Fig. 2 is a partially cut-away sectional view;
Fig. 3 is a sectional view of its main body portion;
Fig. 4 is a longitudinal sectional view showing a second embodiment of the invention;
Fig. 5 is a longitudinal sectional view showing a third embodiment of the invention;
Fig. 6 and Fig. 7 are partially cut-away cross sectional views thereof;
Fig. 8 is a longitudinal sectional view showing a fourth embodiment of the invention;
Fig. 9 to Fig. 11 are partially cut-away cross sectional views thereof;
Fig. 12 is a longitudinal sectional view showing a fifth embodiment of the invention;
Fig. 13 is an explanatory view showing conveying direction of deposit;
Fig. 14 is a longitudinal sectional view showing a sixth embodiment of the invention;
Fig. 15 is a longitudinal sectional view showing a seventh embodiment of the invention; and
Fig. 16 is a longitudinal sectional view showing the seventh embodiment of the invention.

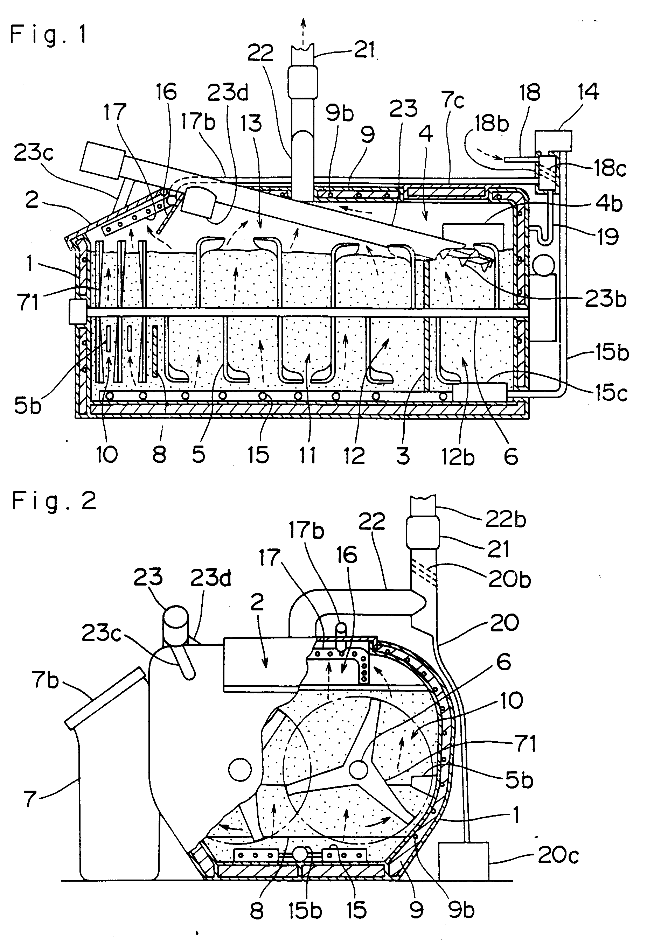

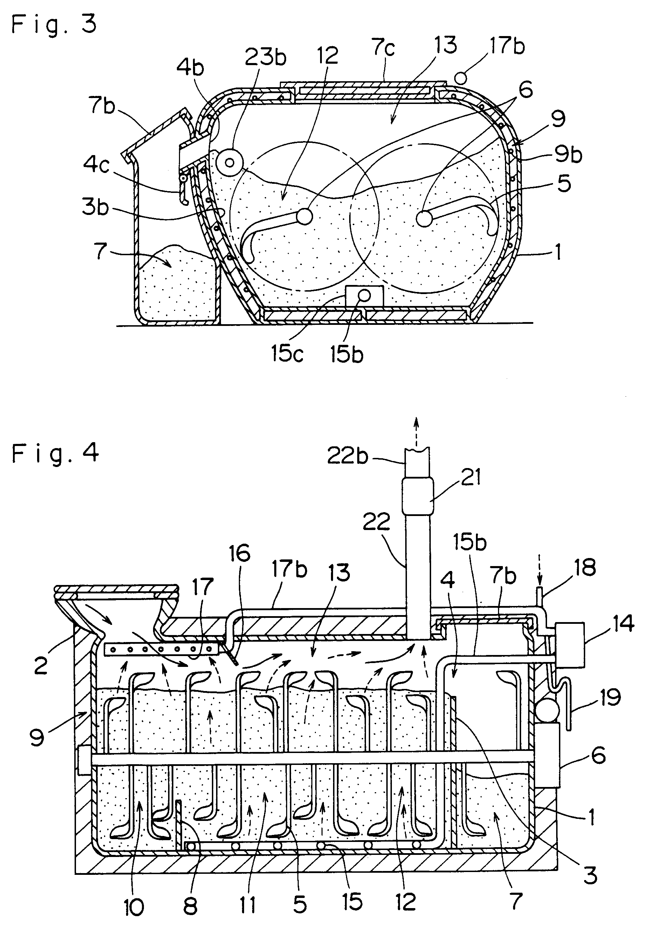

The first embodiment of the invention is described by reference to Figs. 1 to 3. In the upper part of one end side of a container 1, a charging port 2 with a detachable lid is provided. In the container 1 remote from the charging port 2, a partition wall 3 is provided. A rotary drive shaft 6 is horizontally installed in the container 1. This drive shaft 6 possesses multiple agitation pawls 5, 71, and the deposit in the container 1 is uniformly agitated, and functions to move from the charging port 2 side to the partition wall 3 side. As a result, a pre-fermentation region 10, a main fermentation region 11, and a ripening region 12 are continuously formed. The upper part of the partition wall 3 is an overflow port 4, and mature matter overflows from the ripening region 12, and another ripening region 12b is formed. The agitation pawls 5, 71 are intermittently rotated by, for example, a timer. As shown in Fig. 2 and Fig. 3, the drive shaft 6 consists of two parallel shafts.

At the side wall of the ripening region 12b, a discharge port 4b is disposed nearly at the same height as the upper edge of the partition wall 3. On the other hand, at the outside of the container 1, as shown in Fig. 3, a pool 7 is detachably installed, and the mature matter overflowing from the discharge port 4b is stored. A take-out lid 7b is attached to the pool 7. Numeral 4c is a detachable lid of the discharge port 4b. Numeral 7c is a detachable lid of the container 1.

Above the deposit in the container 1, an upper space 13 is formed.

In the pre-fermentation region 10 shown in Fig. 2, a receiving blade 5b is provided on the side wall, and cutting force is strengthened by arranging multiple agitation pawls 71 and receiving blades 5b.

Fig. 3 shows a section of the apparatus in the ripening region 12b, and a small number of agitation pawls 5 powerful in mixing capacity are installed. The main fermentation region 11 is also enhanced in the mixing capacity by a small number of agitation pawls 5.

Nearly in the entire area of the bottom of the container 1, ventilation tubes 15 are installed, and the ventilation tubes 15 coupled with the discharge port of an air pump 14 through a tube duct 15b (see Fig. 2), and air is sent into the container 1.

Beneath the charging port 2, a slant partition board 16 is installed, and the organic refuse charged from the charging port 2 is dropped into the pre-fermentation region 10. Also beneath the charging port 2, an aspiration tube 17 is disposed, and the smell generated by the pre-fermentation region 10 is aspirated. The aspiration tube 17 is coupled to the suction port of the air pump 14 through a tube duct 17b and an airtight box 18b. The airtight box 18b possesses an intake tube 18 for taking in fresh air, aside from the suction port. Beneath the intake tube 18 in the airtight box 18b, a demister 18c (or a mist trap) is disposed, and a U-shaped water-sealed water discharge tube 19 is coupled to the lower end, thereby discharging the condensed water to outside.

In this way, the smell mainly generated from the pre-fermentation region 10 is sucked from the aspiration tube 17, and the smell hardly escapes outside if the charging port 2 is open. The aggregated water in the tube duct 17b is captured in the demister 18c, and is discharged from a short U-tube of the discharge tube 19 because the air resistance is slight. On the other hand, the air containing odor components passes through the stacked layer (main fermentation region 11 and ripening regions 12, 12b) from the ventilation tubes 15, while the odor components are digested by microorganisms by active fermentation, and come out into the upper space 13. The peripheral wall of the container 1 is held in high temperature state by insulator 9 and heating element 9b (electric heating wire, etc.), and the air of high humidity coming out into the upper space 13 does not condense dew, and is aspirated into an exhaust tube 22 provided in the upper part of the container 1 as shown in Fig. 2. The condensed water cooled and formed in the exhaust tube 22 is captured by a demister 20b, and is sent into a water tank 20c through the discharge tube 20. At this time, the slight odor remaining in the exhaust is dissolved and captured in the condensed water, and is hardly released into the atmosphere through a stack 22b.

For efficient fermentation treatment, it is preferred to return a specified amount of mature matter from the ripening region 12b into the pre-fermentation region 10. In this embodiment, accordingly, a screw type returning device 23 is used, and the inlet 23b of the returning device 23 is located near the peripheral wall of the container 1 lower than the discharge port 4b (see Fig. 3), and a first outlet 23d is installed in the middle of the returning device 23, and a second outlet 23c at the front end of the returning device 23. The returning device 23 operates for a specified time only by a timer or the like, and a part of the mature matter just before being discharged from the ripening region 12b and discharge port 4b, and conveyed backward, and the moisture is adjusted first by dropping from the first outlet 23d to the front part of the main fermentation region 11, and the remaining slight amount is dropped from the second outlet port 23c into the pre-fermentation region 10 to be used for seeding.

An auxiliary partition board 8 is provided between the pre-fermentation region 10 and main fermentation region 11, and free water from the pre-fermentation region 10 flows out to the middle of the main fermentation region 11, so that the amount of return to the first outlet 23d can be reduced.

The operation of the embodiment is described below.

A slight amount of prepared mature matter is put into the container 1, and the organic refuse is charged from the charging port 2 to start operation, the air getting into the intake tube 18 from the air pump 14 is supplied in to the stacked layer through the ventilation tubes 15, and the stacked layer is agitated by the agitation pawls 5, 71 in every specified time, and is fermented actively. Furthermore, the stacked layer is agitated uniformly and gradually increases the stacking height to reach a specified height, when it is separated into a pre-fermentation region 10 containing immature organic matter of high moisture content beneath the charging port 2, and ripening regions 12, 12b composed only of sufficiently mature deposit at a remote side from the charging port 2. Between the pre-fermentation region 10 and ripening region 12, there is formed a main fermentation region 11 in which the organic refuse undergoes changes by active fermentation.

The malodor is mainly caused by organic acids generated by chemical composition of immature inorganic refuse in the pre-fermentation region 10 by fermentation temperature. From the main fermentation region 11, ammonia and others may be generated depending on the fermentation condition, but the quantity is slight. The ripening regions 12, 12b are almost odorless, and abundant microorganisms are in malnutrition state.

The malodor generated in the pre-fermentation region 10 is aspirated from the upper aspiration tube 17 in the pre-fermentation region 10 by the air pump 14, and is mixed with the fresh air aspirated from the intake tube 18, and is pumped into the ventilation tubes 15 installed in almost all area of the bottom of the stacked layer including the ripening regions 12, 12b, and sent into the stacked layer from the ventilation tubes 15. At this time, the malodor is digested by the microorganisms while passing through the ripening regions 12, 12b. Therefore, smell is hardly contained in the air aspirated from the upper space 13 by the blower 21 and discharged outside the container 1. A part of the air by the air pump 14 is consumed by passing through the pre-fermentation region 10, but since the excess air ratio at appropriate ventilation amount (the ratio of supply air volume to theoretical minimum air volume necessary for fermentation) is about 20, and hence the oxygen is not sufficient.

When using the returning device 23, in the initial stage of operation, dry organic materials such as grass pellets are contained in the ripening region 12b divided by the partition wall 3, and the organic materials are supplied into the charging port 2 side depending on the charging amount of the organic refuse, and the moisture of the deposit in the main ripening region 11 is adjusted. As the fermentation is progressed and when the height of the stacked layer increases, the mature matter overflows from the partition wall 3,and deposits in the ripening region 12b. As a result, the mature matter is replaced with the dry organic materials, and the deposit in the ripening region 12b is completely occupied by the mature matter, and flows out from the discharge port 4b, and flows into the pool 7. Hence, by accurately setting the returning amount, it functions smoothly into deodorizing action.

The upper space 13 in the container 1 is filled with air high in temperature and saturated in humidity after passing through the stacked layer. When this air is aspirated into the exhaust tube 22 by the blower 21, it is cooled by the fresh air to form a mist flow. By capturing the condensed water by the demister 20b, it is discharged from the exhaust tube 22 into the discharge tube 20. At this time, the majority of the smell remaining in the condensed water, and does not escape into the atmosphere together with the exhaust, so that the deodorizing effect is heightened.

At this time, when the outer circumference of the container 1, especially the upper space 13 is insulated with the insulator 9 and maintained at high temperature by the heating element 9b, the air comes out from the stacked layer to the upper space 13 at high temperature, and flows into the exhaust tube 22 without condensing dew. Accordingly, the moisture carrying amount from within the container 1 is heightened, and the treating capacity is increased.

Besides, the air aspirated from the aspiration tube 17 is cooled while passing through the tube duct 17b which is not insulated, and the condensed water is captured and removed by the demister 18c, and is mixed with fresh air, and ventilated again.

The second embodiment of the invention is described with reference to Fig. 4. The fermentation treatment plant of the embodiment is a small simplified apparatus applied in a household toilet, in particular. In this embodiment, a charging port 2 is a toilet stool with a lid, and a pool 7 is divided by a partition wall 3 in a container 1, and a take-out lid 7b is provided in the upper part. An auxiliary partition board 8 closely contacts with the bottom of the container 1 at its bottom, and the lower part of a pre-fermentation region 10 is a water sump, which is separated from a main fermentation region 11.

The main fermentation region 11 and a ripening region 12 are continuous, and an aspiration tube 17 is provided in the lower part of the charging port 2 surrounding the charging port 2 so as to be aspirated by an air pump 14 through the tube duct 17b. A discharge tube 19 is branched from the end near the air pump 14 of the tube duct 17b, and condensed water is discharged. At the same end, an intake tube 18 is also disposed.

Since this apparatus is small and simplified, and does not comprise the airtight box 18b, demister 20b and returning device 23 as shown in Figs. 1 to 3. The action of the returning device 23 is replaced by the mixing action in the horizontal direction formed by agitation pawls 51 in the longitudinal direction and lateral direction.

The smell is mainly generated in the pre-fermentation region 10, but is aspirated into the aspiration tube 17 as indicated by broken line arrow, and is supplied again in the stacked layer through the air pump 14 and ventilation tube 15, and is deodorized. The air in the upper space 13 slightly containing smell is sucked by a blower and exhausted to a high place through a stack 22b, together with the air entering through the charged port 2.

The same members as in Figs. 1 to 3 are identified with same reference numerals, and their explanations are omitted.

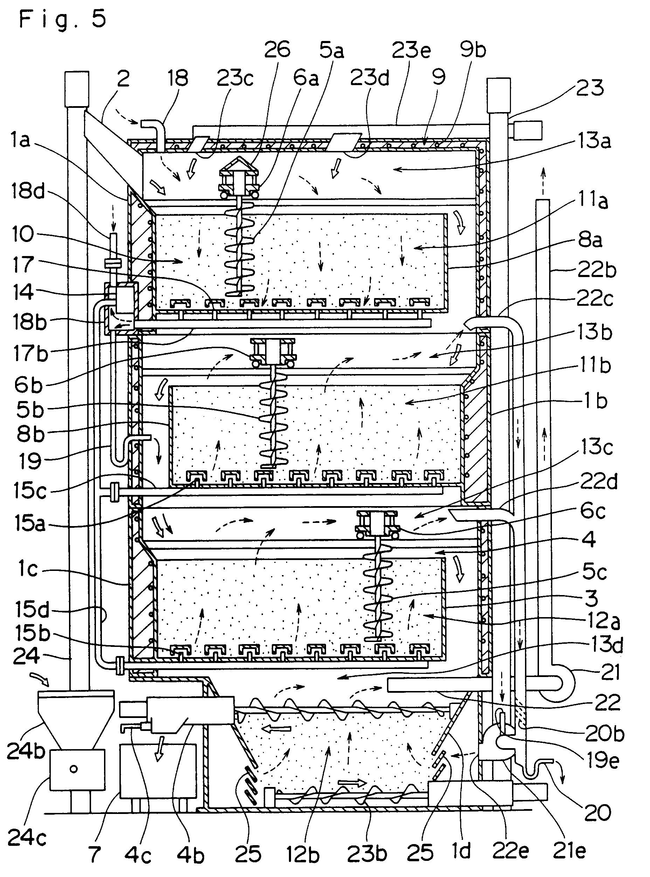

The third embodiment of the invention is described below while referring to Figs. 5 to 7. The fermentation treatment apparatus of the invention is suited to mass treatment of organic refuse in food processing plant, hotel, etc, and in particular suited to mass treatment in a narrow land area.

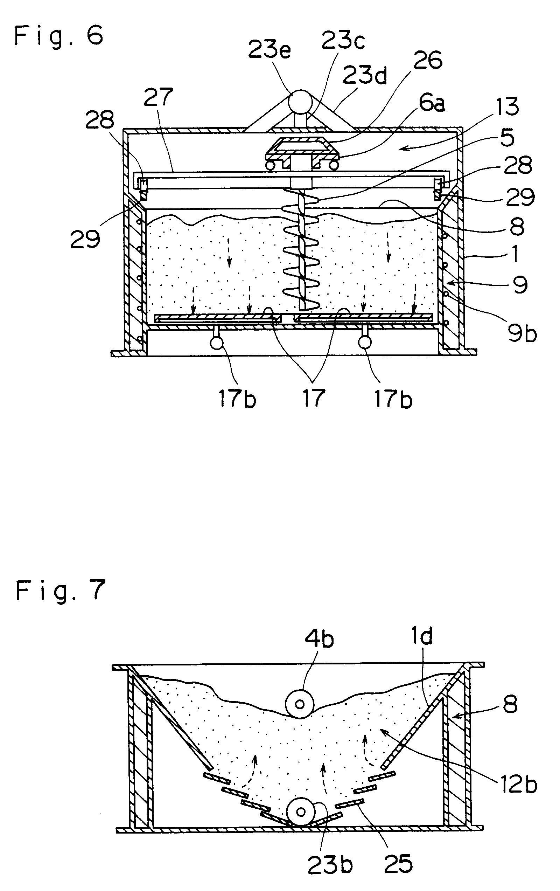

Fig. 5 shows the outline of a four-story apparatus, Fig. 6 shows a cross section of the highest layer, and Fig. 7 is a cross section of the lowest layer.

Sequentially from the highest floor, a first container la, a second container 1b, a third container 1c, and a fourth container 1d are formed, and upper spaces 13a to 13d are provided in the containers 1a to 1d. The outer circumference of the apparatus is insulated with insulator 9 and heating element 9b. In the containers 1a to 1c, agitation pawls 5a to 5c of vertical shaft screw type are provided, and the driving device is supported by carriages 6a to 6c moving longitudinally and laterally on horizontal tracks. That is, as shown in Fig. 6, the carriage 6a can move on a rail 27 elevated in the widthwise direction, and wheels 28, 28 provided at both ends of the rail 27 move on rails 29, 29 elevated in the lengthwise direction of the container 1a. The carriages 6b, 6c are similarly composed.

The upper part of the carriage 6a of the highest layer is covered with a slant plate 26. The first container la and second container 1b possess respectively auxiliary partition walls 8a, 8b, and the third container 1c possesses a partition wall 3. Accordingly, the deposit in each container overflows the individual partition walls 8a, 8b, 3 by agitation, and sequentially drops and moves to the lower containers.

In the bottom of the container 1, plural aspiration tubes 17 are provided, and the aspiration tubes 17 are coupled to the suction port of an air pump 14 through a tube duct 17b and an airtight box 18b. In the stacked layer in the first container 1a, a pre-fermentation region 10 is formed in the charging port 2a, and a part lla of the main fermentation region is formed at the auxiliary partition wall 8a side.

In the lower part of the feed lift 24 coupling to the charging port 2 of the container 1a, a hopper 24b and a grinder 24c are installed, and the charged material is broken into small pieces, and supplied into the container 1a in the elevated place.

In the bottom of the container 1b, plural ventilation tubes 15a are provided, and a main fermentation region 11b is formed in the container 1b. Similarly, in the bottom of the container 1c, plural ventilation tubes 15b are provided, and a ripening region 12a is formed in the container 1c. The upper part of the partition wall 3 of the container 1c is an overflow port 4, through which the mature mater is dropped and collected in the container 1d in the lowest layer. To the ventilation tubes 15a, 15b, tube ducts 15c, 15d connected to the discharge port of the air pump 14 are connected, and air containing smell is sent into the main fermentation region 11b and ripening region 12a.

The inside of the container 1d of the lowest layer is the ripening region 12b, and a screw conveying machine with a shielding plate 4c is provided at the discharge port 4b, and the stacked layer height is kept constant. In the upper space 13d of the container 1d, an aspiration tube 22 of a blower 21 is provided to discharge into the atmosphere through a stack 22b. On the other hand, in the bottom of the container 1d, ventilation plates 25 are provided, and a discharge port of an auxiliary blower 21e is opened. At the suction side of the auxiliary blower 21e, aspiration tubes 22c and 22d opening at the other end are linked to the upper space of the containers 1b and 1c. As a result, the air containing some smell reaches the ripening region 12b through the ventilation plates 25, and is digested and deodorized by microorganisms, and an odor-free air is sucked into the blower 21, and released into the atmosphere from the stack 22b.

Furthermore, in the bottom of the container 1d, a screw type discharge machine 23b is provided, and a returning device 23 for conveying the mature matter in the vertical direction (for example, screw type conveyor) is connected to this screw type discharge machine 23b, and it is further connected to a horizontal transfer machine 23e in the upper part. In the horizontal transfer machine 23e, a charging port 23c for seeding for returning the mature matter into the pre-fermentation region in the container 1a, and a charging port 23d for adjusting the moisture to return the mature matter into the main fermentation region 11a are provided.

The final product is transferred to a conveying truck 7 by opening the shielding plate 4c. Instead of the conveying truck 7,it may be a storage chamber.

In this embodiment, malodor mainly occurs in the container 1a on the highest layer, and it is sucked through the aspiration tube 17 provided in the bottom of the container la together with the air getting in from the intake tube 18. At this time, water formed in the pre-fermentation region 10 also flows into the aspiration tube 17, and air and liquid are separated in the airtight box 18b, and the air is passed and deodorized in the main fermentation region 11b and ripening region 12a through ventilation tubes 15a and 15b. The separated water is discharged into the lower container 1c through a long U-tube 19, thereby reactivating the fermentation.

The containers 1a to 1c are insulated by an insulator 9, and in particular the upper spaces 13b, 13c are held at high temperature by a heating element 9b, and the evaporated moisture is not condensed, and is sucked into the aspiration tubes 22c and 22d together with ventilation, and is cooled by fresh air, and the condensed dew water is captured by a demister 20b, and is discharged through a discharge tube 20. At this time, the majority of odor components is dissolved in water and removed. The air further being rid of moisture is sent into the ripening region 12b through the ventilation plates 25, and is finally deodorized. The mature matter in the ripening region 12b is dried by slight fermentation heat. The air passing through the ripening region 12b is discharged by the blower 21.

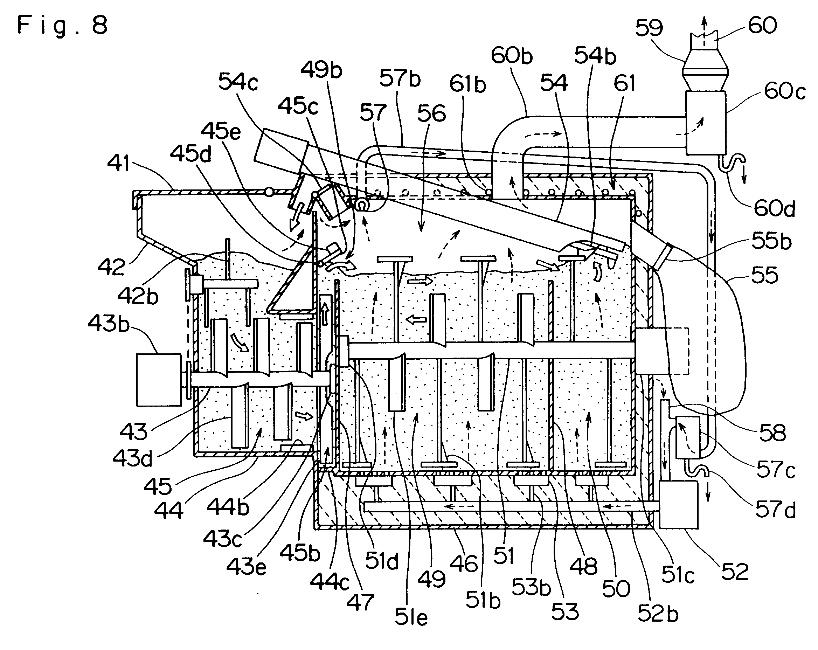

The fourth embodiment of the invention is described with reference to Figs.8 to 11. The fermentation treatment apparatus of the invention is particularly designed to treat waste food discharged from restaurant, food processing factory, and general household.

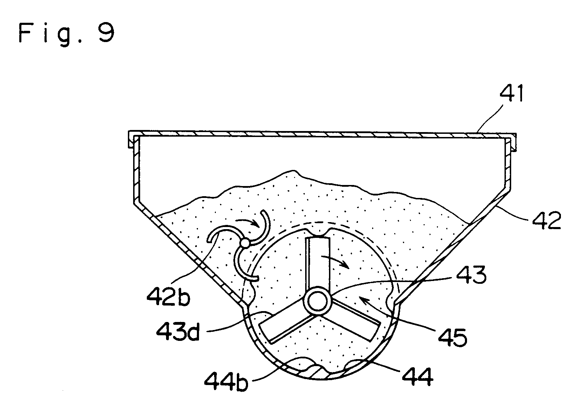

In Fig. 8 and Fig. 9, at the lower end of a charge box 42 possessing a charging port 41 having a detachable lid for charging organic waste, a friction cylnider 44 is connected. A rotary shaft 43 penetrates through the friction cylinder 44 horizontally in its middle. One end of the rotary shaft 43 is supported by a motor 43b, and the other end by a bearing 43c. On the rotary shaft 43, plural twisted pawls 43d for transferring the deposit of the organic waste to the bearing 43c side are planted. In part of the inside of the friction cylinder 44, a baffle 44b is disposed, and a grinding chamber 45 is composed by the rotary shaft 43 and friction cylinder 44.

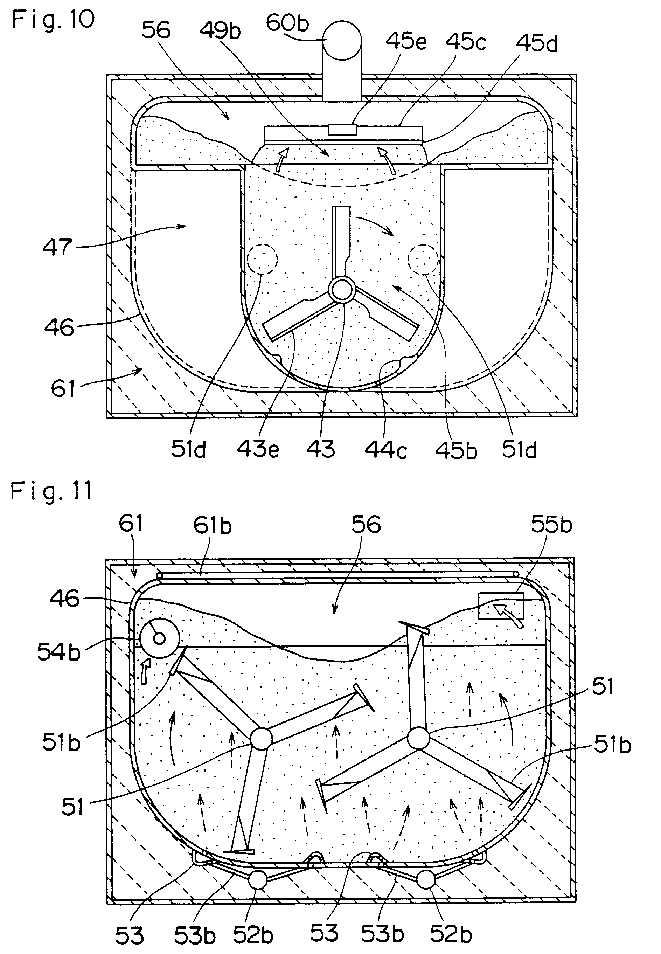

As shown in Fig. 8, a flat pawl 43c is disposed in a portion of the rotary shaft 43 projecting from the grinding chamber 45. As shown in Fig. 10, the pawl 43e is contained in a semicircular secondary friction cylinder 44c, and a secondary grinding chamber 45b is formed. The secondary grinding chamber 45b is enclosed, but an opening 49b communicating with a main fermentation chamber 49 is formed in the upper part. The opening 49b is openably disposed with a hinge 45d, and is closed with a pressurized lid 45c provided with a spindle 45e. The pressurized lid 45c is pushed to open by a pushing pressure of pawls 43d, 43e, and the deposit is moved to the main fermentation chamber 49. By such pressurized lid 45c, conveying amount and grinding action of the deposit are adjusted.

Besides, as shown in Fig. 9, an agitation rotor 42b is installed in the charging box 42 to assist flow of material into the opening 49b of the rotary shaft 43.

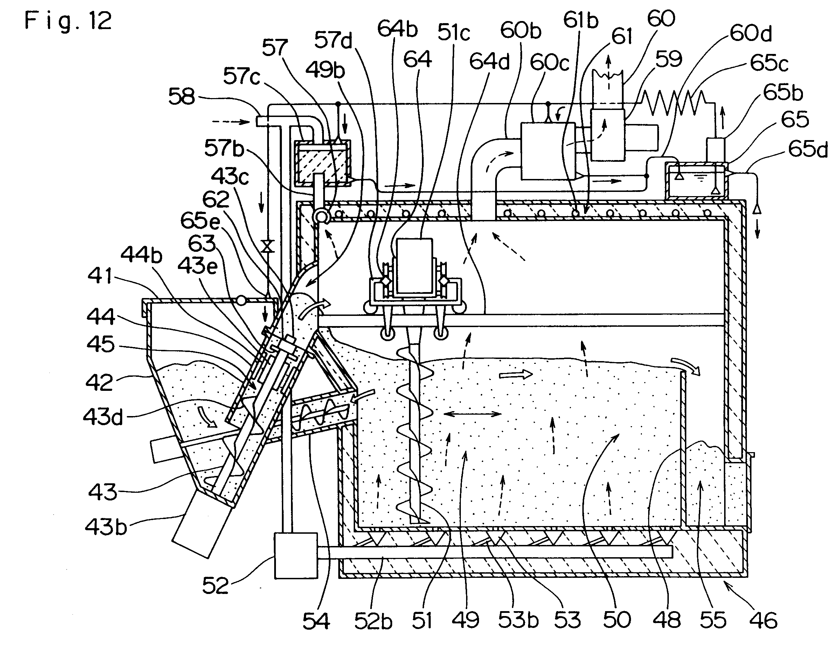

As shown in Fig. 8, the container 46 is partitioned into the secondary grinding chamber 45b, main fermentation chamber 49, and ripening chamber 50 by a front partition board 47 and a rear partition board 48, and an upper space 56 is formed in the upper part. As shown in Fig. 11, in the main fermentation chamber 49 and ripening chamber 50, two parallel agitation shafts 51 extending in the lengthwise direction nearly over the entire length are installed, and agitation pawls 51b, 51e are attached to these agitation shafts 51. One end of each shaft 51 is supported by the front partition board 47 with a bearing 51d, and the other end by a motor 51c mounted on the wall of the container 46, and it is set by a timer so as to rotate intermittently in mutually opposite directions, for example, as indicated by thick line arrow for about 30 minutes in every four hours.

In the lower surfaces of the main fermentation chamber 49 and ripening chamber 50, a multiplicity of ventilation distribution tubes 53 linked to an air pump 52 with ventilation tubes 52b are installed via equalizing fine tubes 53b, so as to ventilate the chambers 49, 50 uniformly.

The front partition board 47 also supports the shaft 43c, and forms an opening 49b of the secondary grinding chamber 45b in the upper part.

A returning device 54 incorporating a screw is provided in the container 46. An inlet 54b of the returning device 54 is disposed at the ripening chamber 50, and the outlet is designed to be opened by changeover operation of a damper 54c into either the charging box 42 or the opening 49b.

A twisted surface is formed on the front end of the agitation pawls 51b, and it functions to transfer the deposit at a position remote from the shaft 51 to the ripening chamber 50 side while agitating the deposit as indicated by double arrow in Fig. 8. The other agitation pawls 51e are shorter than the agitation pawls 51b, and the agitation pawls 51e transfer the deposit near the shaft 51 in the reverse direction of the transfer direction by the agitation pawls 51b, and create convection of the deposit, thereby mixing sufficiently. Accordingly, the returning amount of mature matter by the returning device 54 is decreased, and the moving speed in the main fermentation chamber 49 is decreased to deepen the degree of ripening.

Part of the sufficiently reduced mature matter is returned from the inlet 51b of the returning device 54, and the remainder overflows, as shown in Fig. 11 from a discharge port 55b provided in the upper wall of the container 46 into a pool 55.

As indicated by broken line arrow in Fig. 8 and Fig. 11, the ventilation from the ventilation distribution tubes 53 provided in the bottom of the container 46 passes through the stacked layer, and goes out to the upper space 56 at high temperature and saturated humidity, but since the deposit around the front partition board 47 is immature, malodor is generated. Accordingly, including the odor from the charging box 42, the air in the upper space is aspirated by an aspiration tube 57 as indicated by broken line arrow, and discharged outside of the container 46 by communication tube 57b, and sufficiently cooled by fresh air to condense dew. In succession, the condensed dew water is removed by a demister 57c, and water is discharged from a U-trough 57d. On the other hand, the air being rid of the condensed dew water is mixed with fresh air from an intake tube 58, and passed again into the stacked layer from the tube 53 by the air pump 52. Therefore, the ventilation around the front partition board 47 circulates, but the air ratio is sufficiently large, and it is not inconvenient for fermentation, and by setting the ratio of fresh air from the intake tube 58 is normally set at about 2/3, the odor from the aspiration tube 57 is digested by the microorganisms while passing through the other ripened stacked layer, so that only the exhaust with less odor is released to the atmosphere from an exhaust tube 60 by an exhaust fan 59 through the upper space 56.

To prevent loss of fermentation heat, the entire container 46 is covered and insulated with an insulator 61, and since the exhaust is at high temperature and saturated humidity, a heating element 61b such as electric heating wire is installed in the upper space 56 to prevent dew condensation in the container 46. On the other hand, outside the container 46, a slightly descending long cooling tube 60b, a demister 60c, and a U-trough 60d are installed, and the condensed dew water in which odor components such as ammonia are dissolved is sufficiently condensed, separated, and discharged, and odor-free air is exhausted into the atmosphere.

The operation of this embodiment is explained.

The domestic waste charged into the charging box 42 from the charging port 41 by opening the lid falls and deposits in the grinding chamber 45, and the material tissues are ground and crushed in the compaction condition by the sliding action by high speed rotation of the rotary shaft 43, and the specific surface area is efficiently increased. By the pressurized lid 45c, the compaction condition in the grinding chamber 45 is automatically adjusted, and proper grinding treatment is executed.

Instead of the pressurized lid 45c or together with it, the inside of the friction cylinder 44 may be gradually narrowed, or a curved passage may be formed, which may cause flow resistance and satisfy the compaction condition. Or, by disposing a receiving blade (for example, agitation rotor 42b) in the friction cylinder 44, a part of the friction cylinder 44 is closed to increase the flow resistance in the grinding chamber 45, and then long fibers and livestock animal bones are chopped by a cutter.

The ground material is pushed out of the grinding chamber 45 and charged into the main fermentation chamber 49, and mixed with the mature matter already collected in the main fermentation chamber 49 by agitation shafts 51b, 51e to be adjusted to a proper moisture, so that porous deposit is formed. This deposit has a large reaction surface area, and active bacteria deposit abundantly, generate heat and elevate in temperature by drying and decomposition by more active fermentation by adequate ventilation from the ventilation distribution tubes 53, so that the ventilation carries and discharges a large volume of evaporated moisture. The deposit is sequentially transferred from the main fermentation chamber 49 to the ripening chamber 50 by the action of the agitation shafts 51, and it is similarly ventilated in the ripening chamber to further promote fermentation.

On the other hand, by the action of agitation shafts 51b, 51e, part of the mature deposit near the partition wall 48 in the main fermentation chamber 49 is returned, and mixed with ground material, and hence fermentation is encouraged. Therefore, the transfer of mature matter from the main fermentation chamber 49 to the ripening chamber 50 is very slow, and the ash rate of the deposit increases in the process, and the moisture rate drops, and hence the quantity is extremely reduced, to about 1/50 of the initial charged material volume, and only excess deposit overflows into the pool 55.

Sine the returning device 54 is disposed between the ripening chamber 50 to the charging box 42, and a specified amount of sufficiently fermented mature matter is returned and mixed with the material. However, in the case of material with excessive moisture, it is not returned initially, for example, vegetable refuse is insufficient in the thermal capacity of drying and decomposition as compared with the water content, it is desired to discharge free water as far as possible at the lower end of the grinding chamber 45 to be purified and treated separately. In general food debris, it is preferred to pass the free water from the lower end of the grinding chamber 45 to the main fermentation chamber 49, and save the mixing amount of the mature matter. In cooked rice grains, the water content is not so high as to form free water, and rice grains are hardly disintegrated by agitation but form sticky lumps, and in such a case by sufficiently mixing the mature matter, it is easy to form a porous condition. It is also desired to mix mature material from the beginning in fish debris or the like.

In such a case, by high rate mixing of mature matter of low moisture content, the moisture condition for fermentation is insufficient, and it is adjusted simultaneously by adding water. Therefore, an optimum operation state is selected depending on the type of charged material or moisture rate.

The outlet of the returning device 54 is also opened to the main fermentation chamber 49 by the damper 54c, and it is possible to select to return the mature matter whether to the charging box 42 side or main fermentation side 49 side depending on the material conditions.

The ventilation from the ventilation distribution tubes 53 into the stacked layer is elevated in temperature by fermentation heat, carries a large amount of evaporation moisture to discharge into the upper space 56 of the stacked layer. At this time, the entire container is sufficiently insulated, and the upper space 56 is heated by the heating element 61b, and the exhaust leaving the upper space in saturated state is sucked into the exhaust tube 60b without condensing dew, and in the process of passing through the exhaust tube, the exhaust is cooled and condensed to form a mist flow, and and further in the demister 60c packed with filler, it is cooled as required by sprinkling water to condense dew by force, and water is discharged. In this process, most of odor components are dissolved in water and discharged.

Generally, in the fermentation treatment apparatus of organic refuse, a stimulating malodor is generated by immature matter in the charging box 42 or near the opening 49b, and an aspiration tube 57 is disposed nearby to suck the malodor, and cooled outside the container same as above, and the condensed dew water is discharged by the demister 57c. The dehumidified air is mixed with fresh air in the air pump 52, and passed from the ventilation distribution tubes 53 into the stacked layer, and the odor is digested by the microorganisms in active state. As a result, about one third of the components relatively strong in smell are digested by re-circulation into the stacked layer, and about two thirds weak in smell are sucked into the exhaust tube 60b as mentioned above, to be deodorized and treated.

The fresh air to be mixed in is about 2/3 of the ventilation volume, but the air ratio to the deposit is sufficiently large, and there is no problem for aerobic fermentation. On the other hand, the exhaust from the container is slight, and the odor components are in saturated state, and it is hence preferred to discharge out of the container after precisely executing the deodorizing treatment.

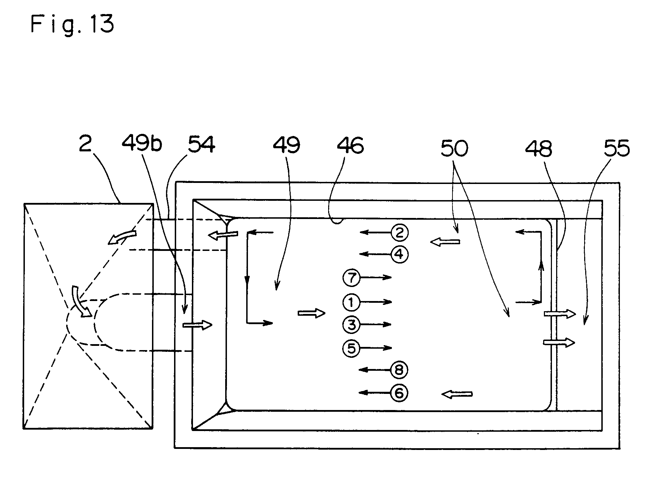

The fifth embodiment of the invention is described by reference to Fig. 12 and Fig. 13. Same members as those shown in Fig. 8 are identified with same reference numerals and explanations are omitted. In the fermentation treatment apparatus in this embodiment, a friction tube 44 is disposed obliquely from the lower end portion of a charging box 42 located at a low position, and a conveying shaft 43 is installed in this friction tube 44. Inside the tube 44, a grinding chamber 45 is composed, and a twisted pawl 43d is composed of a continuous screw pawl. In the installation area of a baffle 44b, a flat pawl 43e with a strong agitation action is installed, and a receiving blade 62 for closing about one third of the section of the tube 44 is fixed and disposed to support a bearing 43c at the front end side of the shaft 43. A cutting blade 63 rotating in contact with the receiving blade 62 is mounted on the shaft 43.

The material charged into the charging box 42 is pushed up by the twisted pawl 43d, and is compacted by the flow resistance by the receiving blade 62. In the baffle 44b part, the material is violently crushed and destroyed in particular, and long fibers are chopped by the cutting blade 63, and they are charged into the vessel 46 through the opening 49b. In the opening 49b, a pressurized lid 45c as shown in the foregoing embodiment may be provided as the means of adjusting action.

Inside the container 46 there is provided an agitation shaft 51 of vertical shaft screw type of small diameter held by a motor 51c. The motor 51c is mounted on a truck 64, and this truck 64 is mounted movably in the widthwise direction of the container 46 on rails 64b. The rails 64b are mounted on other truck 57d, and the truck 57d is installed movably in the longitudinal direction of the container 46 on the rails 64d fixed on the wall of the container.

For example, when the agitation shaft 51 is moved in the sequence of numerals 1 to 8 in Fig. 13, the charged material from the opening 49b is mixed with the deposit in the container 46 by the agitation shaft 51, and is actively fermented by the ventilation from plural ventilation distribution tubes 53 disposed in the lower part of the container 46. As the agitation shaft 51 moves, in the container 46, the deposit moves to the rear partition board 48 side in the middle part as indicated by double arrow in Fig. 13, and is ripened sufficiently, and is returned at both sides of the container 46 to be mixed with new material. As a result, a main fermentation region 49 is formed in the middle of the opening 49b side in the container 46, and ripening regions 50 in the middle part of the rear partition board 48 side and both sides. In the ripening regions 50, the excess mature matter overflows into the pool 55 over the rear partition board 48.

On the basis of the moving track shown in Fig. 13, by further extending the moving range in the longitudinal direction of the container 46 to the overall length to intersect with the track to shorten the moving range in the widthwise direction, it is preferable because the middle part and both side parts are mixed properly. In such case, too, the both sides form ripening regions 50.

Besides, as shown in Fig. 12 and Fig. 13, there is a returning device 54 for returning the deposit from the container 46 to the charging box 42.

The relation between ventilation and exhaust is same as in the preceding embodiment shown in Fig. 8, but in this embodiment a water storage tank 65 is provided. The water in the water storage tank 65 is sprinkled into demisters 60c and 57c through cooling pipe 65c by a water pump 65b, exhaust is cooled and condensation is promoted, and the odor is also captured in the sprinkling water. The discharge water returns into the water storage tank 65 through a U-shaped trough 60d. The excess water is discharged from the water distribution pipe 65d, but in the case of the food debris or other charged material, if the mixing amount of dry mature matter from the returning device 54 is abundant, it is desired to introduce the water in the tank by opening the cock of the water feed port 65e.

In this way, since water is cooling medium, the cooling pipes 60b, 57b may be short, and the effects of deodorization and dehumidification of exhaust are enhanced.

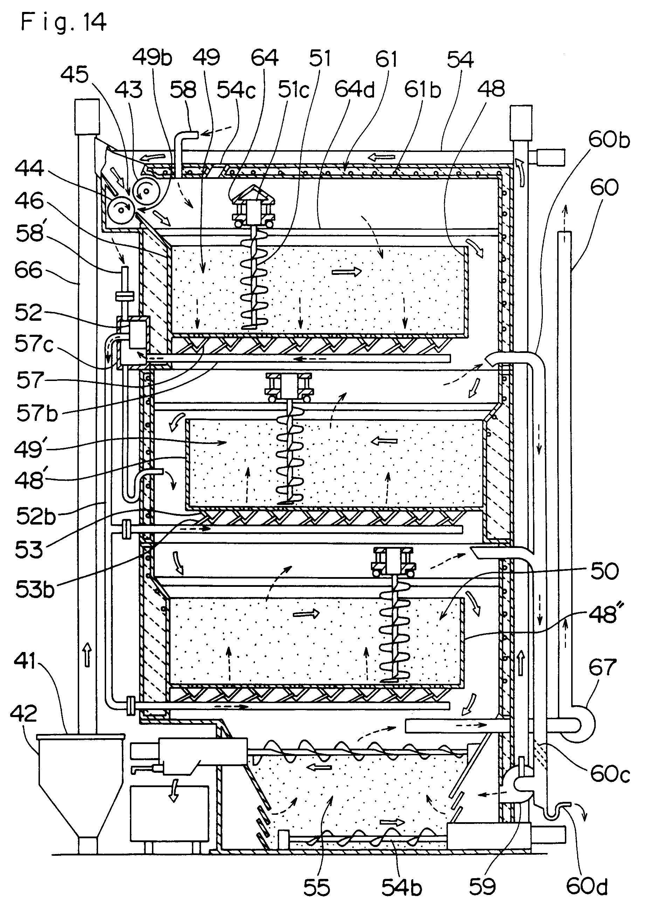

The sixth embodiment of the invention is described by referring to Fig. 14. In this embodiment, the container 46 is in laminate form, and an opening 49b is formed at a high place. Accordingly, to delivery the material from a charging box 42 disposed on the ground, a lift 66 such as conveyor is provided. Before the opening 49b, there is a grinding chamber 45 composed in the gap between two rollers 43 and 44, and one roller 43 moves the material, and the other roller 44 plays the roll of friction cylinder. That is, by rotating these rollers 43 and 44 at different peripheral speeds, the material is compacted and ground while being transferred.

In the lower part of the grinding machine 45, an opening 49b is formed, and crushed material is dropped into a main fermentation chamber 49. In each container 46, an agitation shaft 51 of vertical axis type movable in the widthwise direction and longitudinal direction same as in the previous embodiment is provided.

Overflowing the rear partition board 48 and flowing from the main fermentation chamber 49 through main fermentation chamber 49' and ripening chamber 50, the dry mature matter is sent into the pool 55 in the lowest layer. In the bottom of the pool 55, a screw shaft 54b for returning is provided, and the mature matter from the pool 55 is returned by the returning device 54, and is selectively mixed into the inlet portion of the grinding chamber 45 or the opening 49b portion of the main fermentation chamber 49 by the damper 54c.

The relation between the ventilation and exhaust is nearly same as in the embodiment in Fig. 12, but it is designed to aspirate the odor from the highest layer and ventilate and digest to the lower layers. By cooling the exhaust, and removing the condensed mist, the mature matter in the ripening chamber 55 is passed through the stacked layer, and the odor is finally digested by the microorganisms,and discharged to the atmosphere by other blower 67.

It is preferred to cool the exhaust by using water as medium by installing a tank as shown in Fig. 12. The others are same as shown in Fig. 8, and the same reference numerals are given and explanations are omitted.

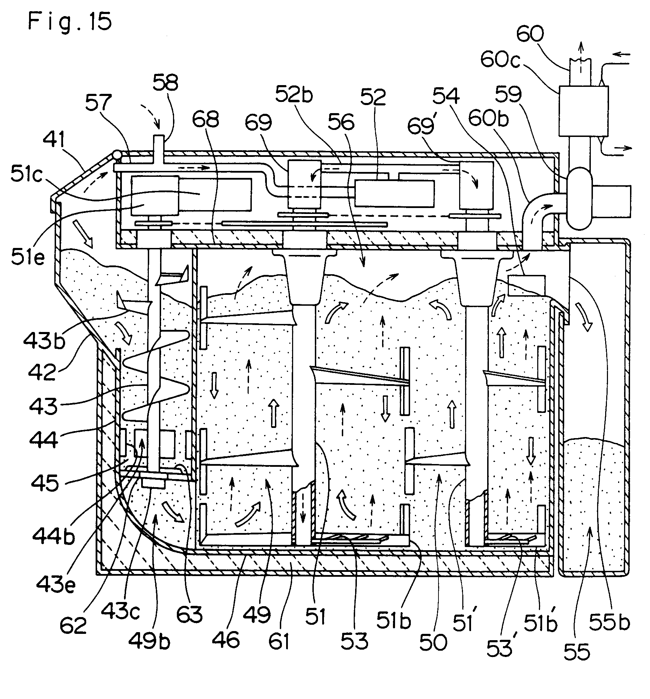

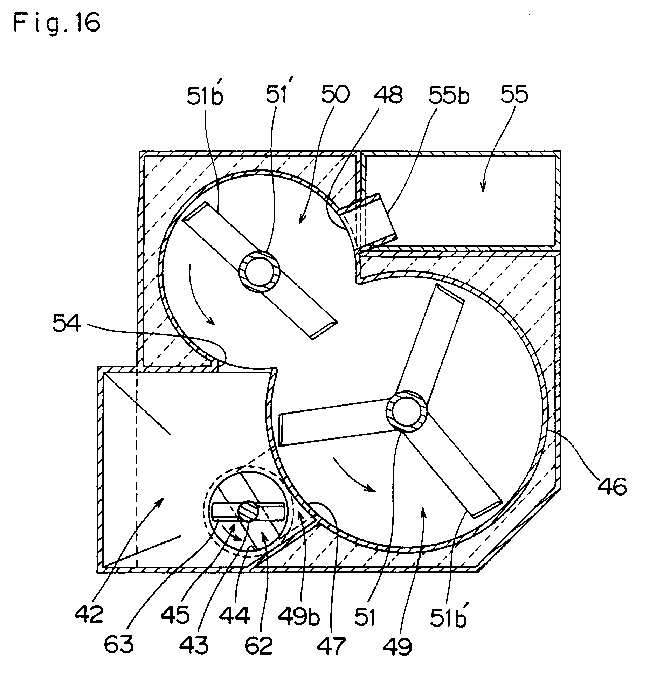

The seventh embodiment of the invention is described below by reference to Fig. 15 and Fig. 16. In this embodiment, a conveying shaft 43 is installed on vertical axis with respect to a charging box 42, and a conveying shaft 43 penetrates into a friction cylinder 44 connected to the lower end of the charging box 42, and a grinding chamber 45 is composed downward. The opening 49b forms a bent tube duct.

On the conveying shaft 43, same as in the embodiment shown in Fig. 12, a cutting blade 63 is mounted on the shaft 43, and a lower end side bearing 43c of the shaft 43 is supported by a receiving blade 62. The conveying shaft 43 is directly coupled with a bevel gear box 51e from a motor 51c, and independent agitation shafts 51, 51' are respectively held on a main fermentation chamber 49 and a ripening chamber 50, by a frame plate 68 forming the upper surface of a container 46, so as to reduce in speed and transmit from the conveying shaft 43 through chain or the like. Agitation shafts 51, 51' are composed of hollow tubes, and their upper ends are coupled to an air pump 52 through sliding shafts 69, 69', and agitation pawls 51b, 51b' incorporating ventilation distribution pipes 53, 53' are provided further at their lower ends, so as to ventilate from the lower surface of the stacked layer while the agitation pawls 51, 51' rotate.

The conveying shaft 43, and agitation shafts 51, 51' are disposed as shown in Fig. 16, and a front partition board 47 is formed between the grinding chamber 45 and main fermentation chamber 49, and an opening 49b is positioned in its lower part. A storage chamber 55 is a detachable box. The mature matter overflows from a discharge port 55b provided in the upper wall of the container 46 of the ripening region 50 side, and stored in the storage chamber 55.

As shown in Fig. 16, the wall of the container 46 of the positioning side of the storage chamber 55 composes a rear partition board 48. The ripening region 50 and charging box 42 are adjacent to each other, and they are coupled with a short return tube 54.

By the rotary action of the twisted pawls 51b, 51b' provided in the agitation shafts 51, 51', the deposit moves from the main fermentation chamber 49 to the ripening chamber 50 while forming convection vertically as indicated by double arrow in Fig. 15, and ripening progresses. At this time, the rotary region of the pawls 51b, 51b' overlaps partly as shown in Fig. 16, and part of the mature matter is returned to the opening 49b is mixed with the ground material. The necessary amount of mature matter is returned from the tube 54 to the charging box 42 side, and the excess overflows from the discharge port 55b into the storage chamber 55.

The others are almost same as in the embodiment shown in Fig. 8, and are identified with same reference numerals, and the explanations are omitted.

## Claims

1. A fermentation treatment apparatus for organic refuse comprising:
(a) an enclosed container (1,46) having;
(i) a charging port (2,41) from which organic refuse is fed to form a stacked layer in said container (1,46) and
(ii) a discharge port (4b,55b) spaced apart from said charging port;
(b) an agitating means for laterally transferring said stacked layer, while agitating the stacked layer vertically, from said charging port (2,41) to the discharge port (4b,55b) in said container (1,46), to continuously form a main fermentation region (11,51) and a ripening region (12,50); and
(c) an exhaust means (22, 22c, 22d, 60c) for exhausting gas generated in said stacked layer outside said container (1,46) through an upper space (13,56); characterised by
(d) a stack height-holding means for holding a height of said stacked layer to form said upper space (13, 56) in said container (1, 46); and
(e) an air supply means on a bottom of said container (1, 46) for supplying air to said stacked layer.

2. A fermentation treatment apparatus for organic refuse as claimed in claim 1, wherein said stack height-holding means comprises the discharge port (4b, 55b) arranged in a wall of said container (1, 46), said stack discharge port (4b, 55b) being spaced apart from said charging port (2, 41) and located at a predetermined height from a bottom of said container (1, 46).

3. A fermentation treatment apparatus for organic refuse as claimed in claim 1, wherein said stack height-holding means comprises a partition wall (3, 48) whose upper part has an overflow port (4), said partition wall (3, 48) being arranged in said container (1, 46) and laterally spaced apart from said charging port (2, 41).

4. A fermentation treatment apparatus for organic refuse as claimed in any of claims 1 to 3, further comprising a returning means (23, 54) for returning a part of the mature matter within said ripening region (12, 50) to said stacked layer in said charging port (2, 41).

5. A fermentation treatment apparatus for organic refuse as claimed in any of claims 1 to 4, comprising sucking and ventilating means having:
an aspiration tube (17) for sucking odorant components generated in said stacked layer in a vicinity of said charging port (2, 41);
a ventilating means for ventilating said odorant components to said stacked layer, and
a gas pumping means (14) for transferring said odorant components from said aspiration tube (17) to said ventilating means.

6. A fermentation treatment apparatus for organic refuse as claimed in any of claims 1 to 5, wherein a demister (20b, 60c) for separating mist in exhaust gas is provided in the exhaust means (22, 22c, 22d, 60).

7. A fermentation treatment apparatus for organic refuse as claimed in any of claims 1 to 6, wherein the container (46) comprises;
a grinding chamber (45) for grinding said organic refuse fed from said charging port (2, 41);
a fermentation and ripening chamber (100) for fermenting and ripening said organic refuse ground by said grinding chamber (45); and
a transferring means for transferring said ground organic refuse from said grinding chamber (45) to said fermentation and ripening chamber (100).

## Patentansprüche

1. Fermentationsbehandlungsvorrichtung für organischen Abfall, die folgendes umfaßt:
(a) Einen geschlossenen Behälter (1, 46) mit:
(i) Einer Beschickungsöffnung (2, 41), aus der organischer Abfall zum Bilden einer gestapelten Schicht in genanntem Behälter (1, 46) gespeist wird und
(ii) einer Austrittsöffnung (4b, 55b), die von genannter Beschickungsöffnung mit Zwischenraum angeordnet ist;
(b) ein Rührmittel zum seitlichen Befordern von genannter gestapelter Schicht, während die gestapelte Schicht vertikal gerührt wird, aus genannter Beschickungsöffnung (2, 41) an die Austrittsöffnung (4b, 55b) in genanntem Behälter (1, 46) zum kontinuierlichen Bilden eines Hauptfermentationsbereichs (11, 51) und eines Reifungsbereichs (12, 50) und
(c) ein Abzugsmittel (22, 22c, 22d, 60c) für Abgas, das in genannter gestapelter Schicht außerhalb genannten Behälters (1, 46) erzeugt wird, durch einen oberen Raum (13, 56), gekennzeichnet durch
(d) ein die Stapelhöhe haltendes Mittel zum Halten einer Höhe von genannter gestapelter Schicht zum Bilden von genanntem oberen Raum (13, 56) in genanntem Behälter (1, 46) und
(e) ein Luftzuleitungsmittel auf einem Boden von genanntem Behälter (1, 46) zum Zuleiten von Luft an genannte gestapelte Schicht.

2. Fermentationsbehandlungsvorrichtung für organischen Abfall nach Anspruch 1, worin genanntes die Stapelhöhe haltendes Mittel folgendes umfaßt: Die Austrittsöffnung (4b, 55b), die in einer Wand von genanntem Behälter (1, 46) angeordnet ist, wobei genannte Stapel-Austrittsöffnung (4b, 55b) mit Zwischenraum von genannter Beschickungsöffnung (2, 41) angeordnet ist und sich in einer vorbestimmten Höhe von einem Boden von genanntem Behälter (1, 46) befindet.

3. Fermentationsbehandlungsvorrichtung für organischen Abfall nach Anspruch 1, worin genanntes, die Stapelhöhe haltendes Mittel eine Trennwand (3, 48) umfaßt, deren oberer Teil eine Überlauföffnung (4) aufweist, wobei genannte Trennwand (3, 48) in genanntem Behälter (1, 46) und seitlich mit Zwischenraum von genannter Beschickungsöffnung (2, 41) angeordnet ist.

4. Fermentationsbehandlungsvorrichtung für organischen Abfall nach einem der Ansprüche 1 bis 3, die überdies ein Rückführmittel (23, 54) zum Rückführen eines Teils des ausgereiften Stoffes in genanntem Reifungsbereich (12, 50) an genannte gestapelte Schicht in genannter Beschickungsöffnung (2, 41) umfaßt.

5. Fermentationsbehandlungsvorrichtung für organischen Abfall nach einem der Ansprüche 1 bis 4, die Absaug- und Lüftungsmittel umfaßt mit:
Einem Absaugrohr (17) zum Absaugen geruchstragender Komponenten, die in genannter gestapelter Schicht in einer Umgebung von genannter Beschickungsöffnung (2, 41) erzeugt werden;
einem Lüftungsmittel zum Lüften genannter geruchstragender Komponenten an genannte gestapelte Schicht und
einem Gaspumpmittel (14) zum Befördern genannter geruchstragender Komponenten aus genanntem Absaugrohr (17) an genanntes Lüftungsmittel.

6. Fermentationsbehandlungsvorrichtung für organischen Abfall nach einem der Ansprüche 1 bis 5, worin ein Nebelabscheider (20b, 60c) zum Abscheiden von Nebel in Abgas in den Abzugsmittel (22, 22c, 22d, 60) vorgesehen ist.

7. Fermentationsbehandlungsvorrichtung für organischen Abfall nach einem der Ansprüche 1 bis 6, worin der Behälter (46) folgendes umfaßt:
Eine Mahlkammer (45) zum Mahlen von genanntem organischem Abfall aus genannter Beschickungsöffnung (2, 41);
eine Fermentations- und Reifungskammer (100) zum Fermentieren und Reifen von genanntem organischem Abfall, der durch genannte Mahlkammer (45) zermahlen wird und
ein Beförderungsmittel zum Befördern von genanntem gemahlenem organischem Abfall aus genannter Mahlkammer (45) an genannte Fermentations- und Reifungskammer (100).

## Revendications

1. Appareil de fermentation de déchets organiques comprenant :
(a) une cuve fermée (1, 46) ayant :
(i) un orifice de chargement (2, 41) par lequel des déchets organiques sont introduits de manière à former une couche empilée dans ladite cuve (1, 46) et
(ii) un orifice de décharge (4b, 55b) espacé dudit orifice de chargement ;
(b) des moyens d'agitation pour transférer latéralement ladite couche empilée, tout en agitant la couche empilée verticalement, dudit orifice de chargement (2, 41) à l'orifice de décharge (4b, 55b) dans ladite cuve (1, 46), de manière à former en continu une zone de fermentation principale (11, 51) et une zone de maturation (12, 50) ; et
(c) des moyens d'échappement (22, 22c, 22d, 60c) pour dégager le gaz généré dans ladite couche empilée à l'extérieur de ladite cuve (1, 46) à travers un espace supérieur (13, 56) ; caractérisé par
(d) des moyens de maintien de la hauteur d'empilement destinés à maintenir une hauteur de ladite couche empilée afin de former un espace supérieur (13, 56) dans ladite cuve (1, 46) ; et
(e) des moyens d'amenée d'air dans une partie inférieure de ladite cuve (1, 46) pour fournir de l'air à ladite couche empilée.

2. Appareil de fermentation de déchets organiques selon la revendication 1, dans lequel lesdits moyens de maintien de la hauteur d'empilement comprennent l'orifice de décharge (4b, 55b) agencé dans une paroi de ladite cuve (1, 46), ledit orifice de décharge d'empilement (4b, 55b) étant espacé dudit orifice de chargement (2, 41) et situé à une hauteur prédéterminée au-dessus du fond de ladite cuve (1, 46).

3. Appareil de fermentation de déchets organiques selon la revendication 1, dans lequel lesdits moyens de maintien de la hauteur d'empilement comprennent une paroi de séparation (3, 48) dont la partie supérieure a un orifice de trop-plein (4), ladite paroi de séparation (3, 48) étant agencée dans ladite cuve (1, 46) et espacée latéralement dudit orifice de chargement (2, 41).

4. Appareil de fermentation de déchets organiques selon l'une quelconque des revendications 1 à 3, comprenant en outre des moyens de retour (23, 54) afin de renvoyer une partie de la matière mûrie dans ladite zone de maturation (12, 50) vers ladite couche enpilée dans ledit orifice de chargement (2, 41).

5. Appareil de fermentation de déchets organiques selon l'une quelconque des revendications 1 à 4, comprenant des moyens d'aspiration et de ventilation ayant :
un tube d'aspiration (17) destiné à aspirer les composants odorants générés dans ladite couche empilée à proximité dudit orifice de chargement (2, 41) ;
des moyens de ventilation destinés à ventiler lesdits composants odorants vers ladite couche empilée, et
des moyens de pompage de gaz (14) destinés à transférer lesdits composants odorants dudit tube d'aspiration (17) vers lesdits moyens de ventilation.

6. Appareil de fermentation de déchets organiques selon l'une quelconque des revendications 1 à 5, dans lequel un dispositif antibuée (20b, 60c) destiné à séparer la vapeur dans le gaz d'échappement est prévu dans les moyens d'échappement (22, 22c, 22d, 60).

7. Appareil de fermentation de déchets organiques selon l'une quelconque des revendications 1 à 6, dans lequel la cuve (46) comprend :
une chambre de broyage (45) pour broyer ledit lit de déchets organiques provenant dudit orifice de chargement (2, 41) ;
une chambre de fermentation et de maturation (100) pour fermenter et mûrir lesdits déchets organiques broyés dans ladite chambre de broyage (45) ; et
des moyens de transfert destinés à transférer lesdits déchets organiques broyés de ladite chambre de broyage (45) à ladite chambre de fermentation et de maturation (100).
